Europäisches Patentamt

European Patent Office

Office européen des brevets

(11) Numéro de publication: **0 054 464**
**B1**

(12) **FASCICULE DE BREVET EUROPEEN**

(45) Date de publication du fascicule du brevet: **21.11.84**

(51) Int. Cl.³: **C 07 C 85/11**, C 07 C 85/24, C 07 C 87/60

(21) Numéro de dépôt: **81401905.5**

(22) Date de dépôt: **02.12.81**

(54) Procédé de préparation d'orthotrifluorométhylaniline.

(30) Priorité: **12.12.80 FR 8026367**

(43) Date de publication de la demande:
**23.06.82 Bulletin 82/25**

(45) Mention de la délivrance du brevet:
**21.11.84 Bulletin 84/47**

(84) Etats contractants désignés:
**AT BE CH DE FR GB IT LI LU NL SE**

(56) Documents cités:
**EP-A-0 038 465**
**EP-A-0 039 810**
**DE-A-1 593 265**
**DE-C- 637 318**
**LU-A- 62 645**

(73) Titulaire: **RHONE-POULENC SPECIALITES CHIMIQUES**
**"Les Miroirs" 18, Avenue d'Alsace**
**F-92400 Courbevoie (FR)**

(72) Inventeur: **Disdier, Camille**
**1, rue du Tonkin**
**F-69100-Villeurbanne (FR)**
Inventeur: **Martinaud, Jacques-Pierre**
**7, rue Barreme**
**F-69006-Lyon (FR)**
Inventeur: **Sullivan, John**
**P.O. Box 388**
**Neshanic Station New Jersey 08853 (FR)**

(74) Mandataire: **Cazes, Jean-Marie et al**
**RHONE-POULENC RECHERCHES Service Brevets Chimie et Polymères 25, quai Paul Doumer**
**F-92408 Courbevoie Cedex (FR)**

Courier Press, Leamington Spa, England.

# 0 054 464

## Description

La présente invention a pour objet un procédé de préparation d'orthotrifluorométhylaniline; elle concerne plus particulièrement un procédé de préparation d'orthotrifluorométhylaniline à partir de trifluorométhylbenzène.

Ce composé est industriellement important puisque il est utilisé comme intermédiaire de synthèse de colorants et de composés à activité phytosanitaire.

On connaît dans l'art antérieur des procédés de ce type. C'est ainsi que Kemichi Fukui et collaborateurs décrivent (Chemical Abstracts 1960 4430 b.) la nitration du trifluorométhylbenzène pour obtenir le métanitrotrifluorométhylbenzène qui est converti en métatrifluorométhylaniline. L'acétylation de ce composé par l'acide acétique donne le métaacétylaminotrifluorométhylbenzène dont la nitration fournit le nitro-2 acétylamino-5 trifluorométhylbenzène.

Ce dernier, par hydrolyse, fournit le nitro-2 amino-5 trifluorométhylbenzène. L'élimination du groupement amino se fait par diazotation réductrice, ce qui permet d'obtenir l'orthonitrotrifluorométhylbenzène dont la réduction conduit à l'orthotrifluorométhylaniline.

Le principal inconvénient de ce type de procédé réside essentiellement dans sa mise en oeuvre longue au plan technique. En effet, sept étapes sont nécessaires pour obtenir le produit recherché à partir du trifluorométhylbenzène. D'autre part, la mise en oeuvre de la réaction de diazotation est, comme cela est bien connu, délicate. De plus, des opérations de purification sont nécessaires après la plupart des étapes. Il faut enfin souligner que les rendements sont limités.

On connaît un autre procédé décrit par L. M. YAGUPOLSKY et N. I. MANKO (Chemical Abstracts 1954 8194 e) qui consiste à traiter par l'hypobromite de sodium l'orthotrifluorométhylbenzamide selon la réaction de dégradation d'Hoffman. Un inconvénient de ce type de réaction est bien connu: il réside dans la possibilité de formation lors de la réaction de dégradation de N-bromoamines qui sont des composés instables. D'autre part, la matière première utilisée dans ce procédé est elle-même d'un accès peu aisé au plan industriel.

On voit donc que subsiste dans l'art antérieur le besoin d'un procédé de préparation d'orthotrifluorométhylaniline dont le nombre d'étapes soit compatible avec une bonne économie du procédé et qui présente des caractéristiques de sécurité satisfaisantes.

L'invention de la demanderesse apporte un tel procédé.

La présente invention concerne donc un procédé de préparation d'orthotrifluorométhylaniline caractérisé en ce que dans une première étape on fait réagir du trifluorométhylbenzène avec du chlore gazeux en présence d'un catalyseur de chloration jusqu'à consommation d'au moins 90% du trifluorométhylbenzène, dans une deuxième étape, on effectue la nitration du mélange brut résultant par action d'un mélange d'acide sulfurique-acide nitrique jusqu'à disparition des chlorotrifluorométhylbenzènes formés lors de la première étape, dans une troisième étape après décantation et lavage de la phase organique, on soumet cette dernière à une première hydrogénation sous pression en présence d'un catalyseur d'hydrogénation constitué par du nickel Raney et/ou nickel Raney dopé au chrome en milieu solvant organique jusqu'à disparition complète des nitrochlorotrifluorométhylbenzènes, puis à une deuxième hydrogénation en présence, d'une quantité supplémentaire de nickel Raney et d'au moins une base alcaline et en ce que dans une quatrième étape, on sépare par distillation du milieu réactionnel résultant l'orthotrifluorométhylaniline.

La facilité d'obtention d'orthotrifluorométhylaniline par le procédé ci-dessus est en soi très supprenante. En effet, la chloration pratiquement complète de trifluorométhylbenzène conduit comme cela est connu à l'obtention d'un mélange de dérivés mono et dichlorés. A côté du métachlorotrifluorométhylbenzène obtenu en proportion prépondérante et des dérivés para- et ortho, on constate la présence dans des proportions pouvant atteindre 20—25% des isomères du dichlorotrifluorométhylbenzène.

Cette présence importante de produits dichlorés conduisait tout naturellement l'homme de l'art, soit à les séparer, soit à minimiser leur formation. Le premier cas implique des opérations de distillation qui alourdissent la technique et le coût de fabrication. Dans le deuxième cas, il est nécessaire de diminuer le taux de conversion du trifluorométhylbenzène et, par voie de conséquence, d'introduire une étape de distillation après la chloration pour séparer le trifluorométhylbenzène n'ayant pas réagi.

On retrouve les mêmes inconvénients que ceux évoqués pour le premier cas.

On voit donc que l'homme de l'art se trouvait devant une impasse technique et économique compte tenu du fait que les dérivés dichlorés étaient considérés comme un obstacle à la suite des opérations.

La demanderesse a découvert que, d'une manière surprenante, les composés dichlorés obtenus en fin de première étape pouvaient être considérés comme un élément avantageux dans la mesure où dans la suite du procédé ils favorisent la formation de l'orthotrifluorométhylaniline, produit recherché. En d'autres termes l'élément qui faisait le plus obstacle à l'adoption d'une telle voie par l'homme de l'art a été dégagé par la demanderesse comme étant un élément favorisant à la fois la technique du procédé et son économie.

En effet, le rendement est augmenté et la mise en oeuvre du procédé devient aisé du fait de la non-nécessité des distillations intermédiaires.

2

**0 054 464**

Plus précisément, la demanderesse a constaté que d'une façon surprenante, la nitration des produits dichlorés était réalisable dans les mêmes conditions que celle des dérivés monochlorés. Elle a de plus constaté, ce qui n'était pas non plus établi par l'art antérieur, que la réduction et l'hydro-déchloration des dérivés nitrodichlorés pouvaient s'effectuer dans des conditions aisées.

Selon un mode de réalisation préférentiel de l'invention, le catalyseur de chloration est choisi parmi le groupe comprenant seul ou en mélange: le chlorure ferrique, le pentachlorure d'antimoine et les métaux susceptibles d'engendrer in situ ces deux composés: le fer et l'antimoine.

On préfère plus particulièrement utiliser le pentachlorure d'antimoine et le chlorure ferrique.

On opère de préférence avec une concentration en catalyseur comprise entre 0,1% et 10% en poids par rapport au trifluorométhylbenzène engagé. Encore plus préférentiellement, ce pourcentage est compris entre 0,5 et 5.

La chloration se fait généralement sans solvant bien que l'utilisation d'un solvant organique ne soit pas exclue.

On effectue cette chloration à une température de préférence comprise entre 0 et 100°C et encore plus particulièrement entre 10 et 80°C, sous pression atmosphérique bien que des pressions supérieures ou inférieures à la pression atmosphérique ne soient pas hors du cadre de l'invention.

La conversion du trifluorométhylbenzène est de préférence supérieure à 95%. Elle peut atteindre 100%.

On opère, pour l'étape de nitration sans solvant bien que là aussi l'utilisation de solvant ne soit pas exclue du domaine de l'invention.

Les acides $HNO_3$—$H_2SO_4$ sont utilisés en quantités telles que le rapport pondéral de l'acide sulfurique aux chlorotrifluorométhylbenzènes présents dans le milieu réactionnel est compris de préférence entre 0,5 et 5 et plus particulièrement entre 0,7 et 2; le rapport pondéral de l'acide nitrique aux chlorotrifluorométhylbenzènes est de même compris de préférence entre 0,5 et 5 et plus particulièrement entre 0,7 et 2.

La nitration est effectuée de préférence à une température comprise entre 0 et 80°C et plus particulièrement entre 10 et 60°C, sous pression atmosphérique bien que des pressions différentes ne soient pas, là aussi, exclues du domaine de l'invention.

Le lavage de la phase organique après la réaction de nitration peut se faire par exemple à l'eau et à la soude.

Selon un mode de réalisation préférentiel de l'invention, on effectue la première hydrogénation de la troisième étape sous une pression d'hydrogène comprise entre 10 et 50 bars; plus préférentiellement, on choisit une pression comprise entre 10 et 30 bars.

La température est, de préférence, comprise entre 20 et 100°C et, plus préférentiellement, entre 40 et 80°C.

On effectue cette première hydrogénation en présence, comme catalyseur d'hydrogénation, de nickel Raney et/ou de nickel Raney dopé au chrome. On entend par nickel Raney dopé au chrome, du nickel Raney contenant entre 0,5 et 5% en poids de chrome.

Le catalyseur est utilisé en quantité telle que sa concentration est comprise entre 3 et 10% en poids par rapport aux chloronitrotrifluorométhylbenzènes. Encore plus préférentiellement, ce pourcentage est compris entre 4 et 8%.

Le solvant est choisi de préférence parmi le groupe comprenant le méthanol, l'éthanol et le propanol. On préfère utiliser le méthanol.

La deuxième hydrogénation de la troisième étape est réalisée dans les mêmes conditions de température, de pression d'hydrogène et de solvant que la première. On introduit dans le milieu réactionnel, au moins une mole de base alcaline par mole de chlorotrifluorométhylbenzène initialement présente.

On utilise comme base alcaline, la soude ou la potasse. La soude est plus particulièrement préférée.

On introduit également en cours de cette deuxième hydrogénation, en une ou plusieurs fois, entre 8 et 20% en poids de nickel Raney par rapport aux chloronitrotrifluorométhylbenzènes initialement présents. Encore plus préférentiellement, ce pourcentage est compris entre 9 et 15%.

La présente invention apparaîtra plus clairement à la lecture des exemples qui vont suivre, qui ne sauraient être considérés comme une limitation quelconque du procédé objet de la présente demande.

Exemple 1

Dans un réacteur équipé d'un agitateur, d'un injecteur de gaz et d'un absorbeur à acide chlorhydrique, on introduit 29,2 kg de trifluorométhylbenzène et 292 g de pentachlorure d'antimoine. On introduit ensuite du chlore gazeux à 20°C pendant 8 heures. Après lavage à l'eau, on obtient 35,340 kg d'un mélange dont l'analyse par chromatographie en phase vapeur révèle qu'il contient:

— 4% de trifluorométhylbenzène
— 70,6% de métachlorotrifluorométhylbenzène
— 5,8% de parachlorotrifluorométhylbenzène
— 3% d'orthochlorotrifluorométhylbenzène
— et 16,6 de dichlorotrifluorométhylbenzènes (3 isomères)

3

Le taux de conversion du trifluorométhylbenzène est de 95% et le rendement global est de 96%.

Dans un réacteur agite, on introduit 15,940 kg d'acide sulfurique concentré (97%) et 15,940 kg d'acide nitrique fumant.

On refroidit à 10°C ce mélange et on introduit, sous agitation à cette température et en 1 heure, 18,05 kg du mélange brut précédemment obtenu de trifluorométhylbenzène, chlorotrifluorométhylbenzènes et dichlorotrifluorométhylbenzènes.

Quand l'introduction est achevée, on porte le mélange à 50°C et on le maintient 6 heures à cette température. On décante la phase organique que l'on lave à l'eau, à la soude à 10% puis encore à l'eau. On recueille ainsi 21,62 kg d'un mélange dont l'analyse par chromatographie en phase vapeur donne la composition suivante:

— chlorotrifluorométhylbenzènes=non dosables
— nitrotrifluorométhylbenzène=3,9%
— chloronitrotrifluorométhylbenzènes=82,2% (4 isomères)
— dichloronitrotrifluorométhylbenzènes=13,9% (4 isomères)

Le taux de conversion des chlorotrifluorométhylbenzènes est de 100%, le rendement global est de 96%.

Dans un autoclave agité, on introduit 1,400 l de méthanol et 50 g de nickel Raney. On porte sous 20 bars d'hydrogène à 20°C et on agite pendant 15 minutes. On introduit ensuite 1,46 l de méthanol et 1 kg de la phase organique précédemment obtenue. On effectue l'hydrogénation à 80°C sous 20 bars. Au bout d'une heure et demi, on constate par chromatographie en phase vapeur que la totalité des chloronitrotrifluorométhylbenzènes a été consommée. On refroidit à 50°C et on introduit 622 g de lessive de soude à 30,8%. On agite 15 minutes sous 20 bars d'hydrogène.

On introduit alors 50 g de nickel Raney et on hydrogène sous 20 bars à 50°C pendant deux heures après lesquelles on introduit encore 50 g de nickel Raney. On poursuit l'hydrogénation pendant encore 2 heures. La réaction est alors achevée. Après filtration concentration et lavage à l'eau, on recueille 684 g d'un mélange d'amines avant la composition suivante:

— orthotrifluorméthylaniline=77,2%
— métatrifluorométhylaniline=20,5%
— paratrifluorométhylaniline=2,3%

On soumet ce mélange à distillation au moyen d'une colonne de 20 plateaux avec un reflux de 29 sous une pression de 50 mm de Hg. On obtient en tête 522 g d'orthotrifluorométhylaniline de titre supérieur à 99% ce qui correspond à un rendement de 99%.

Exemple 2

Dans le réacteur de l'exemple 1, on introduit 7,300 kg de trifluorométhylbenzène, 41 g de chlorure ferrique et 20 g de fer. On introduit ensuite du chlore à 20°C pendant 6 heures. Après lavage à l'eau, on obtient 9160 g d'un mélange dont l'analyse par chromatographie en phase vapeur révèle qu'il contient:

— trifluorométhylbenzène: 1,7%
— métachlorotrifluorométhylbenzène: 66,4%
— parachlorotrifluorométhylbenzène: 6,1%
— orthochlorotrifluorométhylbenzène: 2,6%
— dichlorotrifluorométhylbenzènes: 24,3%

Le taux de conversion du trifluorométhylbenzène est de 98% et le rendement global est de 98%.

Dans un réacteur agité, on introduit 2753 g du mélange brut précédent. On refroidit à 10°C et on introduit sous agitation à cette température et en 1 heure le mélange suivant: acide nitrique fumant (1700 g) et acide sulfurique concentré (97%) (2350 g).

On porte ensuite le mélange à 80°C pendant 6 heures. On décante la phase organique que l'on lave à l'eau, à la soude à 10% puis encore à l'eau. On recueille ainsi 3290 g d'un mélange dont l'analyse révèle la composition suivante:

— chlorotrifluorométhylbenzène: non dosable
— nitrotrifluorométhylbenzène: 1,8%
— chloronitrotrifluorométhylbenzènes: 74,8%
— dichloronitrotrifluorométhylbenzènes: 23,4%

Le taux de conversion des chlorotrifluorométhylbenzènes est de 100%, le rendement global est de 96%.

Dans un autoclave agité, on introduit 2900 ml de méthanol et 50 g de nickel Raney dopé au chrome. On porte sous 12 bars d'hydrogène à 50°C sous agitation pendant 15 minutes. On introduit ensuite dans ces conditions en 1 H 45 mn, 1000 g du produit de nitration précédemment obtenu. On maintient ensuite pendant encore 1 H 30 mn à 50°C sous 12 bars d'hydrogène et sous agitation. On constate alors que la totalité des chloronitrotrifluorométhylbenzènes a été consommée. On introduit alors 600 g de lessive de soude à 31,1% et on agite à 50°C sous 12 bars d'hydrogène pendant 15 minutes. On ajoute ensuite 50 g de Nickel Raney et on hydrogène pendant 2 heures à 50°C sous 12 bars d'hydrogène. On introduit de nouveau 50 g de Nickel Raney et l'on poursuit l'hydrogénation pendant 3 heures dans les conditions précédentes. La réaction est alors achevée.

Après filtration, concentration et lavage à l'eau, on recueille 670 g d'un mélange contenant:

— orthotrifluorométhylaniline: 76,7%
— métatrifluorométhylaniline: 21,2%
— paratrifluorométhylaniline: 2,1%

Ou soumet ce mélange à distillation comme dans l'exemple 1. On obtient 498 g d'orthotrifluorométhylaniline d'un titre supérieur à 99%, ce qui correspond à un rendement de distillation de l'orthotrifluorométhylaniline de 97%.

Exemple 3

Dans le réacteur de l'exemple 1, on introduit 7,300 kg de trifluorométhylbenzène, 62,4 g d'antimoine. On introduit ensuite du chlore à 20°C pendant 3 H 30 mn. Après lavage à l'eau, on obtient 9170 g d'un mélange dont l'analyse par chromatographie en phase vapeur révèle qu'il contient:

— trifluorométhylbenzène: 0,7%
— métachlorotrifluorométhylbenzène: 65,5%
— parachlorotrifluorométhylbenzène: 5,2%
— orthochlorotrifluorométhylbenzène: 1,5%
— dichlorotrifluorométhylbenzènes: 27,1%

Le taux de conversion du trifluorométhylbenzène est de 99,1% et le rendement global est de 97%.

Dans un réacteur agité, on introduit 2753 g du mélange brut précédent. On refroidit à 10°C et on introduit sous agitation à cette température et en 1 heure, le mélange suivant: acide nitrique fumant (1700 g) et acide sulfurique concentré (97%) (2350 g).

On porte ensuite le mélange à 80°C pendant 6 heures. On décante la phase organique que l'on lave à l'eau, à la soude à 10% puis encore à l'eau. On recueille ainsi 3200 g d'un mélange dont l'analyse révèle la composition suivante:

— chlorotrifluorométhylbenzène: non dosable
— nitrotrifluorométhylbenzène: 0,7%
— chloronitrotrifluorométhylbenzènes: 72,9%
— dichloronitrotrifluorométhylbenzènes: 26,4%

Le taux de conversion des chlorotrifluorométhylbenzène est de 100%, le rendement global est de 94%.

Dans un autoclave agité, on introduit 2900 ml de méthanol et 50 g de Nickel Raney dopé au chrome. On porte sous 12 bars d'hydrogène à 50°C sous agitation pendant 15 minutes. On introduit ensuite dans ces conditions en 1 H 45 mn, 1000 g du produit de nitration précédemment obtenu. On maintient ensuite pendant encore 1 H 30 mn à 50°C sous 12 bars d'hydrogène et sous agitation. On constate alors que la totalité des chloronitrotrifluorométhylbenzènes a été consommée. On introduit alors 630 g de lessive de soude à 31,1% et on agite à 50°C sous 12 bars d'hydrogène pendant 15 minutes. On ajoute ensuite 50 g de Nickel Raney et on hydrogène pendant 2 heures à 50°C sous 12 bars d'hydrogène. On introduit de nouveau 50 g de Nickel Raney et on poursuit l'hydrogénation pendant 3 heures dans les conditions précédentes. La réaction est alors achevée.

Après filtration, concentration et lavage à l'eau, on recueille 660 g d'un mélange contenant:

— orthotrifluorométhylaniline: 78,2%
— métatrifluorométhylaniline: 19,7%
— paratrifluorométhylaniline: 2,1%

On soumet ce mélange à distillation comme dans l'exemple 1. On obtient 505 g d'orthotrifluorométhylaniline d'un titre supérieur à 99%, ce qui correspond à un rendement de distillation de l'orthotrifluorométhylaniline de 98%.

**Revendications**

1. Procédé de préparation d'orthotrifluorométhylaniline caractérisé en ce que dans une première étape, on fait réagir du trifluorométhylbenzène avec du chlore gazeux en présence d'un catalyseur de chloration jusqu'à consommation d'au moins 90% du trifluorométhylbenzène, dans une deuxième étape, on effectue la nitration du mélange brut résultant par action d'un mélange acide sulfurique-acide nitrique jusqu'à disparition des chlorotrifluorométhylbenzènes formés lors de la première étape, dans une troisième étape, après décantation et lavage de la phase organique, on soumet cette dernière à une première hydrogénation sous pression en présence d'un catalyseur d'hydrogénation constitué par du nickel Raney et/ou nickel Raney dopé au chrome en milieu solvant organique jusqu'à disparition complète des nitrochlorotrifluorométhylbenzènes, puis à une deuxième hydrogénation en présence d'une quantité supplémentaire de nickel Raney et d'au moins une base alcaline et en ce que dans une quatrième étape, on sépare par distillation du milieu réactionnel résultant l'orthotrifluorométhylaniline.

2. Procédé selon la revendication 1 caractérisé en ce que le catalyseur de chloration est choisi parmi le groupe comprenant seul ou en mélange le chlorure ferrique, le pentachlorure d'antimoine, le fer et l'antimoine.

3. Procédé selon la revendication 2 caractérisé en ce que le catalyseur de chloration est le pentachlorure d'antimoine.

4. Procédé selon l'une quelconque des revendications précédentes caractérisé en ce que la concentration du catalyseur de chloration est comprise entre 0,1 et 10% en poids par rapport au trifluorométhylbenzène engagé.

5. Procédé selon la revendication 1 caractérisé en ce qu'on effectue la première étape à une température comprise entre 0 et 100°C sous pression atmosphérique.

6. Procédé selon la revendication 1 caractérisé en ce qu'on effectue la première étape jusqu'à consommation d'au moins 95% de trifluorométhylbenzène.

7. Procédé selon la revendication 1 caractérisé en ce que dans la deuxième étape, l'acide sulfurique et l'acide nitrique sont utilisés en quantités tel que le rapport pondéral de chacun d'eux aux chlorotrifluorométhylbenzènes présents dans le milieu réactionnel est compris entre 0,5 et 5.

8. Procédé selon la revendication 1 caractérisé en ce que la deuxième étape est mise en oeuvre à une température comprise entre 0 et 80°C sous pression atmosphérique.

9. Procédé selon la revendication 1 caractérisé en ce que dans la troisième étape, on utilise une pression d'hydrogène comprise entre 10 et 50 bars.

10. Procédé selon la revendication 1 caractérisé en ce que la concentration du catalyseur d'hydrogénation lors de la première hydrogénation est comprise entre 3 et 10% en poids par rapport aux chloronitrotrifluorométhylbenzènes.

11. Procédé selon la revendication 1 caractérisé en ce que, dans la troisième étape, le solvant est choisi parmi le groupe comprenant le méthanol, l'éthanol et le propanol.

12. Procédé selon la revendication 1 caractérisé en ce que le solvant est le méthanol.

13. Procédé selon la revendication 1 caractérisé en ce que dans la troisième étape, la température est comprise entre 20 et 100°C.

14. Procédé selon la revendication 1 caractérisé en ce que dans la deuxième hydrogénation de la troisième étape, la base alcaline est la soude ou la potasse.

15. Procédé selon la revendication 1 caractérisé en ce que dans la deuxième hydrogénation de la troisième étape, on introduit en une ou plusieurs fois entre 8 et 20% en poids de nickel Raney par rapport aux chloronitrotrifluorométhylbenzènes initialement présents.

**Patentansprüche**

1. Verfahren zur Herstellung von Orthotrifluormethylanilin, dadurch gekennzeichnet, daß man in einer ersten Stufe Trifluormethylbenzol mit Chlorgas in Anwesenheit eines Chlorierungskatalysators umsetzt, bis mindestens 90% des Trifluormethylbenzols verbraucht ist, in einer zweiten Stufe das resultierende Rohgemisch durch die Einwirkung einer Mischung von Salpetersäure und Schwefelsäure nitriert, bis die in der ersten Stufe entstandenen Chlortrifluormethylbenzole umgesetzt sind, und in einer dritten Stufe nach Dekantieren und Waschen der organischen Phase diese einer ersten Hydrierung unter Druck in Anwesenheit eines Hydrierungskatalysators aus Raney-Nickel und/oder aus mit Chrom dotiertem Raney-Nickel in einem organischen Lösungsmittel bis zur völligen Umsetzung der Nitrochlortrifluormethylbenzole unterwirft, und dann einer zweiten Hydrierung in Anwesenheit einer zusätzlichen Menge an Raney-Nickel und mindestens einer Alkalibase unterwirft, und dadurch, daß man in einer vierten Stufe das Orthotrifluormethylaniline von dem gebildeten Reaktionsgemisch durch Destillation abtrennt.

2. Verfahren nach Anspruch 1, dadurch gekennzeichnet, daß der Chlorierungskatalysator aus der Gruppe von Eisen(III) Chlorid, Antimonpentachlorid, Eisen und Antimon gewählt und allein oder als Mischung dieser Stoffe verwendet wird.

3. Verfahren nach Anspruch 2, dadurch gekennzeichnet, daß der Chlorierungskatalysator Antimonpentachlorid ist.

4. Verfahren nach einem der vorangehenden Ansprüche, dadurch gekennzeichnet, daß die Konzentration des Chlorierungskatalysators zwischen 0,1 und 10 Gew.%, bezogen auf das eingesetzte Trifluormethylbenzol, liegt.

5. Verfahren nach Anspruch 1, dadurch gekennzeichnet, daß man die erste Stufe bei einer Temperatur zwischen 0 und 100°C unter Atmosphärendruck durchführt.

6. Verfahren nach Anspruch 1, dadurch gekennzeichnet, daß man die erste Stufe bis zur Umsetzung von mindestens 95% des Trifluormethylbenzols durchführt.

7. Verfahren nach Anspruch 1, dadurch gekennzeichnet, daß in der zweiten Stufe Salpeter- und Schwefelsäure in einer solchen Menge verwendet werden, daß das Gewichtsverhältnis von jeder der beiden Säuren zu den im Reaktionsgemisch vorhandenen Chlortrifluormethylbenzolen zwischen 0,5 und 5 liegt.

8. Verfahren nach Anspruch 1, dadurch gekennzeichnet, daß man in der zweiten Stufe bei einer Temperatur zwischen 0 und 80°C und Atmosphärendruck arbeitet.

9. Verfahren nach Anspruch 1, dadurch gekennzeichnet, daß man in der dritten Stufe eine Wasserstoffdruck von 10 bis 50 bar anwendet.

10. Verfahren nach Anspruch 1, dadurch gekennzeichnet, daß die Konzentration des Hydrierkatalysators während der ersten Hydrierung zwischen 3 und 10 Gew.%, bezogen auf die Chlornitrotrifluormethylbenzole, liegt.

11. Verfahren nach Anspruch 1, dadurch gekennzeichnet, daß in der dritten Stufe das Lösungsmittel aus der Gruppe von Methanol, Ethanol und Propanol gewählt ist.

12. Verfahren nach Anspruch 1, dadurch gekennzeichnet, daß das Lösungsmittel Methanol ist.

13. Verfahren nach Anspruch 1, dadurch gekennzeichnet, daß man in der dritten Stufe bei einer Temperatur zwischen 20 und 100°C arbeitet.

14. Verfahren nach Anspruch 1, dadurch gekennzeichnet, daß bei der zweiten Hydrierung in der dritten Stufe die Alkalibase Natrium- oder Kaliumhydroxid ist.

15. Verfahren nach Anspruch 1, dadurch gekennzeichnet, daß man bei der zweiten Hydrierung in der dritten Stufe mit einem Mal oder in mehreren Portionen zwischen 8 und 20 Gew.% Raney-Nickel, bezogen auf die ursprünglich vorhandenen Chlornitrotrifluormethylbenzole, zugibt.

**Claims**

1. Process for the preparation of ortho-trifluoromethylaniline, characterised in that in a first stage trifluoromethylbenzene is reacted with gaseous chlorine in the presence of a chlorination catalyst until at least 90% of the trifluoromethylbenzene has been consumed, in a second stage the nitration of the resulting crude mixture is carried out by treatment with a mixture of sulphuric acid and nitric acid until the clorotrifluoromethylbenzenes formed during the first stage have disappeared, in a third stage, after phase separation and washing of the organic phase, the latter is subjected to a first hydrogenation under pressure in the presence of a hydrogenation catalyst consisting of Raney nickel and/or Raney nickel doped with chromium in an organic solvent medium until the nitrochlorotrifluoromethylbenzenes have completely disappeared and thereafter to a second hydrogenation in the presence of an additional amount of Raney nickel and of at least one alkali metal base, and in a fourth stage the ortho-trifluoromethylaniline is isolated by distillation from the resulting reaction mixture.

2. Process according to Claim 1, characterised in that the chlorination catalyst is chosen from the group comprising — individually or as a mixture — ferric chloride, antimony pentachloride, iron and antimony.

3. Process according to Claim 2, characterised in that the chlorination catalyst is antimony pentachloride.

4. Process according to any one of the preceding claims, characterised in that the concentration of the chlorination catalyst is between 0.1 and 10% by weight relative to the trifluoromethylbenzene employed.

5. Process according to Claim 1, characterised in that the first stage is carried out at a temperature between 0 and 100°C, under atmospheric pressure.

6. Process according to Claim 1, characterised in that the first stage is carried out until at least 95% of trifluoromethylbenzene has been consumed.

7. Process according to Claim 1, characterised in that in the second stage the sulphuric acid and the nitric acid are used in such amounts that the weight ratio of each of them to the chlorotrifluoromethylbenzenes present in the reaction mixture is between 0.5 and 5.

8. Process according to Claim 1, characterised in that the second stage is carried out at a temperature of between 0 and 80°C under atmospheric pressure.

9. Process according to Claim 1, characterised in that in the third stage a hydrogen pressure of between 10 and 50 bars is used.

10. Process according to Claim 1, characterised in that the concentration of the hydrogenation catalyst during the first hydrogenation is between 3 and 10% by weight relative to the chloronitrotrifluoromethylbenzenes.

11. Process according to Claim 1, characterised in that in the third stage the solvent is chosen from among the group comprising methanol, ethanol and propanol.

12. Process according to Claim 1, characterised in that the solvent is methanol.

13. Process according to Claim 1, characterised in that in the third stage the temperature is between 20 and 100°C.

14. Process according to Claim 1, characterised in that in the second hydrogenation of the third stage the alkali metal base is sodium hydroxide or potassium hydroxide.

15. Process according to Claim 1, characterised in that in the second hydrogenation of the third stage between 8 and 20% of the Raney nickel, relative to the chloronitrotrifluoromethylbenzenes initially present, is introduced in one or a plurality of stages.